# EUROPÄISCHE PATENTSCHRIFT

(11) **EP 3 110 469 B1**
(45) Veröffentlichungstag und Bekanntmachung des Hinweises auf die Patenterteilung: **14.04.2021**
(21) Anmeldenummer: 15706463.5
(22) Anmeldetag: 24.02.2015
(51) Int. Cl.: A61M 1/14, A61M 1/36

(54) **MEDIZINISCHE FLÜSSIGKEITSKASSETTE UND MEDIZINISCHER SCHLAUCHSATZ MIT MEDIZINISCHER FLÜSSIGKEITSKASSETTE**
MEDICAL FLUID CASSETTE AND MEDICAL TUBE SET COMPRISING A MEDICAL FLUID CASSETTE
CASSETTE DE FLUIDE MÉDICAL ET JEU DE TUYAUX MÉDICAUX COMPRENANT UNE CASSETTE DE FLUIDE MÉDICAL

(30) Priorität: 26.02.2014 DE 102014002578
(43) Veröffentlichungstag der Anmeldung: 04.01.2017
(73) Patentinhaber: Fresenius Medical Care Deutschland GmbH, 61352 Bad Homburg (DE)
(72) Erfinder: GOESSMANN, Stephan, 61381 Friedrichsdorf (DE); HÄCKER, Jürgen, 61267 Neu-Anspach (DE)
(74) Vertreter: Rau, Jenspeter
(86) Internationale Anmeldenummer: PCT/EP2015/053783
(87) Internationale Veröffentlichungsnummer: WO 2015/128306

(56) Entgegenhaltungen:
- WO-A2-2012/017417
- DE-A1-102009 018 664
- US-A1- 2004 127 841
- US-A1- 2010 269 702
- US-A1- 2010 270 230

## Beschreibung

### Technisches Gebiet

Die Erfindung betrifft eine medizinische Flüssigkeitskassette und einen medizinischen Schlauchsatz mit einer medizinischen Flüssigkeitskassette zum Führen und/oder Aufnehmen einer medizinischen Flüssigkeit. Insbesondere betrifft die Erfindung einen Blutschlauchsatz mit medizinischer Flüssigkeitskassette für die extrakorporale Blutbehandlung.

### Hintergrund

Aus dem Stand der Technik sind medizinische Flüssigkeitskassetten zum Führen und/oder Aufnehmen einer medizinischen Flüssigkeit bekannt. Die Schrift WO 2010/121819 A1 offenbart eine Blutkassette für die extrakorporale Blutbehandlung, welche wenigstens einen Gehäusekörper und wenigstens eine in den Gehäusekörper integrierte Kammer zum Führen und/oder Aufnehmen medizinischer Fluide und wenigstens einen in den Gehäusekörper integrierten Kanal zum Führen und/oder Aufnehmen eines medizinischen Fluids aufweist. In der bekannten Blutkassette ist stromabwärts einer in den Gehäusekörper integrierten Luftblasenabscheidekammer in einer weiteren separaten Kammer ein Gerinnselfilter integriert. Der Platzbedarf für diese bekannte Integration des Gerinnselfilters im dem Gehäusekörper limitiert bei gegebenen kompakten Abmessungen der Blutkassette jedoch den verbleibenden Platz für andere im Gehäusekörper zu integrierende Strukturen, wie beispielsweise Kammern zum Führen und/oder Aufnehmen medizinischer Fluide. Bei der Fertigung der Blutkassette wird der bekannte Gerinnselfänger durch Bördeln oder Laserschweißen fest mit dem Gehäusekörper verbunden.

Des Weiteren sind aus dem Stand der Technik Flüssigkeitskassetten bekannt, die als Blasenkammern ausgebildet sind, die in deren Innerem einen zylindrischen oder konischen Gerinnselfilter vor der Austrittsöffnung der Luftblasenabscheidekammer aufweisen. Eine solche Luftblasenabscheidekammer ist beispielsweise aus der Schrift WO 2008/065472 A1 bekannt. Ein Nachteil dieser bekannten Luftblasenabscheidekammer mit integriertem Gerinnselfänger kann in der aufgrund der komplizierten Geometrie aufwändigen Fertigung gesehen werden. Ein weiterer Nachteil kann in dem geringen Verweilvolumen und dem geringen Strömungsquerschnitt stromabwärts der Gerinnselfilteröffnungen gesehen werden.

Die Schrift US 2010/0269702 A1 beschreibt einen Luftblasenabscheider und eine Blutkassette mit Luftblasenabscheider, wobei der Luftblasenabscheider eine erste Kammer mit einem Zufluss und eine zweite Kammer mit einem Abfluss umfasst und stromabwärts vom Abfluss ein Gerinnselfänger angeordnet ist.

Die Schrift US 2010/0270230 A1 beschreibt einen Gerinnselfänger, der eine scheibenförmige Siebfläche zum Auffangen von Gerinnseln in einem die Siebfläche durchströmenden Fluid aufweist.

Die Schrift US 2004/0127841 A1 beschreibt eine Blutkassette mit einem Filterelement für eine Photopherese-Behandlung von Blut.

Die Schrift WO 2012/017417 A2 beschreibt einen Wärmeübertrager für medizinische Flüssigkeiten, in dem eine Kammer durch ein Diaphragma in zwei Bereiche aufgeteilt wird.

Es ist eine Aufgabe der vorliegenden Erfindung eine medizinische Flüssigkeitskassette zum Führen und/oder Aufnehmen einer medizinischen Flüssigkeit bereitzustellen, die zumindest einen der vorgenannten Nachteile oder eine der vorgenannten Limitierungen überwindet.

Es ist eine weitere Aufgabe der vorliegenden Erfindung eine Blutkassette mit integriertem Gerinnselfänger bereitzustellen, die zumindest einen der vorgenannten Nachteile oder eine der vorgenannten Limitierungen überwindet.

Es ist eine weitere Aufgabe der vorliegenden Erfindung Bauteile mit komplizierter Geometrie ohne Bördeln oder Schweißen in eine medizinische Flüssigkeitskassette zu integrieren und damit den Aufwand bei der Fertigung zu verringern.

Diese Aufgaben werden gelöst mit den Merkmalen der unabhängigen Patentansprüche 1 und 7. Vorteilhafte Ausführungsformen sind in den Unteransprüchen angegeben.

Die vorliegende Erfindung stellt eine medizinische Flüssigkeitskassette bereit, aufweisend einen Gehäusekörper, der einen ersten Wandungsabschnitt einer Kammer zum Aufnehmen und/oder Führen einer medizinischen Flüssigkeit ausbildet, eine Abdeckung, die einen zweiten Wandungsabschnitt der Kammer ausgebildet, einen Einsatz mit einem ersten Umfangsabschnitt und einem zweiten Umfangsabschnitt, wobei der Einsatz ausgebildet ist zum zumindest teilweisen Ausfüllen eines Querschnitts der Kammer, wobei der Querschnitt der Kammer zumindest von dem ersten Wandungsabschnitt und dem zweiten Wandungsabschnitt begrenzt wird und wobei der Einsatz die Kammer in einen ersten Kammerabschnitt und einen zweiten Kammerabschnitt unterteilt, und eine Aufnahme in und/oder an dem ersten Wandungsabschnitt, wobei die Aufnahme zum Aufnehmen des ersten Umfangsabschnitts ausgebildet ist und wobei der erste Umfangsabschnitt in der Aufnahme aufgenommen ist.

Der erste Wandungsabschnitt begrenzt zumindest abschnittsweise ein Inneres der Kammer. Der erste Wandungsabschnitt begrenzt die Kammer insbesondere im Bereich der Aufnahme. Der Gehäusekörper kann eine Montagefläche aufweisen, und die Kammer kann als Kavität in der Montagefläche ausgebildet sein, so dass die Kammer in der Montagefläche eine Ausnehmung ausbildet. Die Montagefläche kann zumindest abschnittsweise eine Umrandungskante der Kammer ausbilden und/oder zumindest abschnittsweise über die Umrandungskante hinausgehen. Der zweite Wandungsabschnitt begrenzt zumindest abschnittsweise ein Inneres der Kammer. Insbesondere begrenzt der zweite Wandungsabschnitt die Kammer im Bereich der Aufnahme. Beispielsweise begrenzt die der Kammer zugewandte Oberfläche der Abdeckung zumindest abschnittsweise ein Inneres der Kammer. Die Abdeckung kann die durch die Kammer in der Montagefläche gebildete Ausnehmung und die Umrandungskante der Ausnehmung abdecken und darüber hinaus zumindest abschnittsweise einen Bereich außerhalb der Umrandungskante abdecken. Die Abdeckung kann die Montagefläche somit zumindest teilweise oder sogar vollständig überdecken.

Wenn die Abdeckung als flexible Folie ausgebildet ist, dann begrenzt die der Kammer zugewandte Oberfläche der Folie zumindest abschnittsweise ein Inneres der Kammer. Besonders vorteilhaft wird die Folie zumindest abschnittsweise dauerhaft mit der Montagefläche verbunden. Die Folie kann beispielsweise im Bereich der Umrandungskante und/oder im Bereich außerhalb der Umrandungskante dauerhaft mit der Montagefläche verbunden sein.

Unter einer dauerhaften Verbindung der Abdeckung mit der Montagefläche wird in der vorliegenden Erfindung eine Verbindung verstanden, zu deren Aufrechterhaltung nach dem Verbinden keine weiteren Hilfsmittel benötigt werden.

Die Montagefläche kann zumindest abschnittsweise oder vollständig eben ausgebildet sein.

In speziellen Ausführungsformen kann der Gehäusekörper vorteilhaft durch Spritzgießen oder Thermoformen aus Kunststoff, insbesondere Polypropylen (PP) hergestellt sein. Die Abdeckung kann als flexible Folie aus Kunststoff, insbesondere Polypropylen (PP) ausgebildet sein. In solchen Ausführungsformen kann die Abdeckung zumindest abschnittsweise mit der Montagefläche durch Schweißen oder Kleben dauerhaft verbunden sein.

In manchen Ausführungsformen kann die Abdeckung lose auf dem zweiten Umfangsabschnitt aufliegen, wenn der Einsatz in der Aufnahme eingesetzt ist und die Abdeckung dauerhaft mit der Montagefläche verbunden ist. In solchen Ausführungsformen besteht keine dauerhafte Verbindung zwischen dem zweiten Umfangsabschnitt und dem zweiten Wandungsabschnitt. Der zweite Wandungsabschnitt kann jedoch mit dem zweiten Umfangsabschnitt vorübergehend eine flüssigkeitsdichte Verbindung ausbilden, wenn der zweite Wandungsabschnitt an den zweiten Umfangsabschnitt angepresst wird. Das Anpressen kann beispielsweise bei der bestimmungsgemäßen Verwendung der medizinischen Flüssigkeitskassette zum Aufnahmen und/oder Führen der medizinischen Flüssigkeit erfolgen. Bei Wegfall der Anpresskraft ist die Verbindung nicht mehr flüssigkeitsdicht. Die Anpresskraft wird kann insbesondere aufgebracht werden, wenn die Abdeckung der medizinischen Flüssigkeitskassette an eine Ankoppelfläche einer Behandlungsvorrichtung oder Behandlungsmaschine angekoppelt ist.

In anderen Ausführungsformen kann der zweite Wandungsabschnitt mit dem zweiten Umfangsabschnitt dauerhaft eine flüssigkeitsdichte Verbindung ausbilden. In solchen Ausführungsformen kann der zweite Wandungsabschnitt mit dem zweiten Umfangsabschnitt verschweißt oder verklebt sein, so dass zur Aufrechterhaltung der flüssigkeitsdichten Verbindung keine weiteren Hilfsmittel benötigt werden. Auch bei solchen Ausführungsformen kann die Abdeckung der medizinischen Flüssigkeitskassette an eine Ankoppelfläche einer Behandlungs-vorrichtung oder Behandlungsmaschine angekoppelt werden. Jedoch wird bei dieser Ausführungsform keine spezielle Anpresskraft benötigt, um den zweiten Wandungsabschnitt mit dem zweiten Umfangsabschnitt flüssigkeitsdicht zu verbinden.

Die Aufnahme kann zumindest abschnittsweise als eine Nut und/oder als Rille und/oder als Steg und/oder als Absatz am und/oder im ersten Wandungsabschnitt ausgebildet sein. Die Aufnahme dient einerseits beim Zusammenbau der medizinischen Flüssigkeitskassette dem Aufnehmen und/oder Führen und/oder Halten des ersten Umfangsabschnitts an der bestimmungsgemäßen Position in der Kammer der medizinischen Flüssigkeitskassette. Der Zusammenbau des Gehäusekörpers mit dem Einsatz kann beispielsweise durch Einschieben des Einsatzes in seine Endposition in der Aufnahme erfolgen, bevor die Abdeckung am Gehäusekörper befestigt wird, weil in diesem Fertigungszustand der Gehäuse-körper halboffen und daher von außen zugänglich ist.

Die Aufnahme kann als eine umlaufende Nut und/oder als umlaufende Rille und/oder als umlaufender Steg und/oder als umlaufender Absatz am und/oder im ersten Wandungsabschnitt ausgebildet sein. In solchen Ausführungsformen kann erste Umfangsabschnitt flüssigkeitsflüssigkeitsdicht mit der Aufnahme in Verbindung stehen, wenn der Einsatz in die Aufnahme eingesetzt ist.

In manchen Ausführungsformen liegen alle Abschnitte der Aufnahme in einer Ebene, so dass ein ebener Einsatz eingeschoben werden kann. Dabei ist die Dicke des Einsatzes im Bereich des ersten Umfangsabschnitts im Wesentlichen konstant.

In manchen Ausführungsformen wird die Aufnahme durch zwei Begrenzungs-flächen gebildet, die in zwei zueinander parallele Ebenen liegen, wobei der Raum zwischen den parallelen Ebenen zum Einschieben und/oder Aufnehmen des Einsatzes ausgestaltet ist und wobei die parallelen Ebenen den ersten Wandungsabschnitt und den zweiten Wandungsabschnitt schneiden. Dabei ist die Form des Einsatzes zumindest im Bereich des ersten Umfangsabschnitts zum Einschieben und/oder Aufnehmen des Einsatzes zwischen den zwei Begrenzungsflächen ausgestaltet.

In speziellen Ausführungsformen wird die Aufnahme durch zwei Begrenzungs-flächen gebildet, die in zwei zueinander parallelen Ebenen liegen, wobei der Raum zwischen den parallelen Ebenen zum Einschieben und/oder Aufnehmen des Einsatzes ausgestaltet ist und wobei die parallelen Ebenen den ersten Wandungsabschnitt und/oder den zweiten Wandungsabschnitt unter einem im Wesentlichen rechten Winkel schneiden. Auch bei diesen Ausführungsformen ist die Form des Einsatzes zumindest im Bereich des ersten Umfangsabschnitts zum Einschieben und/oder Aufnehmen des Einsatzes zwischen den zwei Begrenzungsflächen ausgestaltet.

Der zweite Umfangsabschnitt kann gerade ausgeführt sein, wobei dann der erste Umfangsabschnitt zumindest abschnittsweise gekrümmt oder eckig ausgeführt ist.

Der Einsatz kann zumindest eine Öffnungsstruktur aufweisen zum flüssigkeitsdurchlässigen Verbinden des ersten Kammerabschnitts mit dem zweiten Kammerabschnitt.

In speziellen Ausführungsformen kann die Öffnungsstruktur einen Gerinnselfilter aufweisen.

In manchen Ausführungsformen kann der erste Kammerabschnitt und/oder der zweite Kammerabschnitt als Luftblasenabscheider ausgebildet sein.

Ein Vorteil der vorliegenden Erfindung ist, dass das Einsetzen und/oder Einschieben des Einsatzes in die Aufnahme bei der Fertigung der medizinischen Flüssigkeitskassette automatisiert durch einen Montageroboter erfolgen kann. Alternativ wird natürlich auch ein manuelles Einsetzen und/oder Einschieben des Einsatzes in die Aufnahme erleichtert, weil die Aufnahme und der Einsatz fertigungs- und montagegerecht ausgestaltet sind.

In bestimmten Ausführungsformen kann die medizinische Flüssigkeitskassette kompakt ausgebildet sein und trotzdem gleichzeitig zum Messen des Drucks in der medizinischen Flüssigkeit sowohl stromaufwärts des Gerinnselfilters als auch stromabwärts des Gerinnselfilters ausgebildet sein, wobei jeweils der Druck der medizinischen Flüssigkeit auf die flexible Folie messbar ist, indem von außen ein Drucksensor an die Abdeckung angekoppelt ist. Die Messstelle stromaufwärts des Gerinnselfilters kann auch stromaufwärts eines stromaufwärts des Gerinnselfilters angeordneten Luftblasenabscheiders angeordnet sein. Dann ist eine Druck-messung stromaufwärts des Luftblasenabscheiders und stromabwärts des Gerinnselfilters möglich. Dieser Vorteil wird dadurch erreicht, dass der Gerinnselfilter platzsparend als Einsatz in der Kammer ausgebildet ist. Somit steht in der medizinischen Flüssigkeitskassette stromaufwärts des Gerinnselfilters und stromabwärts des Gerinnselfilters Platz für die Druckmessstelle an der flexiblen Folie zur Verfügung, wobei die medizinische Flüssigkeitskassette gleichzeitig kompakt ist.

### Kurze Beschreibung der Zeichnungen

Im Folgenden wird ein Ausführungsbeispiel nach der vorliegenden Lehre unter Bezugnahme auf die Figuren näher erläutert. Anhand des in den Figuren dargestellten Ausführungsbeispiels werden weitere Einzelheiten und Vorteile näher beschrieben. Die Bezugszeichen in den Figuren haben jeweils durchgängig in allen Figuren die gleiche Bedeutung.

Es zeigen:

Figur 1 eine freigeschnittene Ansicht und eine Schnittdarstellung eines Ausschnittes einer erfindungsgemäßen medizinischen Flüssigkeitskassette

Figur 2 eine freigeschnittene perspektivische Ansicht und eine Schnittdarstellung eines Ausschnittes einer erfindungsgemäßen medizinischen Flüssigkeitskassette mit erfindungsgemäßem Einsatz

Figur 3 einen erfindungsgemäßen Einsatz mit Gerinnselfilter für die medizinische Flüssigkeitskassette gemäß Figuren 1 und 2

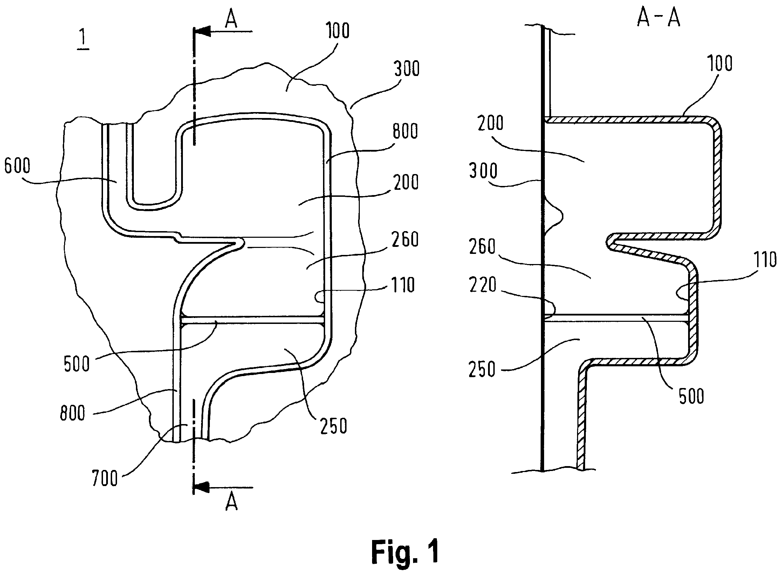

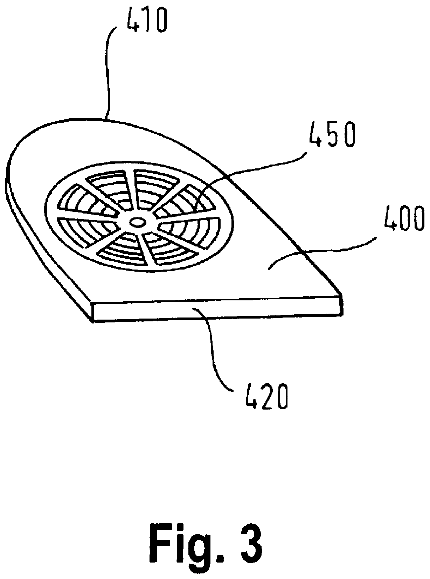

Die **Figur 1** zeigt eine freigeschnittene Ansicht (linke Abbildung) und eine Schnittdarstellung (rechte Abbildung) eines Ausschnittes einer erfindungsgemäßen medizinischen Flüssigkeitskassette (1) mit einem Gehäusekörper (100) und einer in den Gehäusekörper integrierten Kammer (200). Die Kammer (200) weist einen ersten Wandungsabschnitt (110) auf. Der erste Wandungsabschnitt (110) ist Teil des Gehäusekörpers (100) und begrenzt die Kammer (200) insbesondere im Bereich der Aufnahme (500). Der Gehäusekörper (100) weist eine Montagefläche auf, an der die Abdeckung (300), die eine flexible Folie ist, abschnittsweise festgeschweißt ist, und die Kammer (200) ist als Kavität in der Montagefläche ausgebildet, so dass die Kammer (200) in der Montagefläche eine Ausnehmung ausbildet. Die Montagefläche bildet zumindest abschnittsweise eine in einer Ebene liegende Umrandungskante (800) der Kammer (200) aus, wobei die Umrandungskante (800) die Ausnehmung zumindest abschnittsweise umrandet und wobei die Montagefläche zumindest abschnittsweise über die Umrandungskante (800) hinausgeht. Der zweite Wandungsabschnitt (220) ist Teil der Abdeckung (300) und die der Kammer (200) zugewandte Oberfläche der Abdeckung (300) begrenzt zumindest abschnittsweise das Innere der Kammer (200), insbesondere im Bereich der Aufnahme (500). Die Abdeckung (300) deckt die durch die Kammer (200) in der Montagefläche gebildete Ausnehmung und zumindest die Umrandungskante der Ausnehmung vollständig ab. Darüber hinaus deckt die Abdeckung (300) Bereiche der Montagefläche außerhalb der Umrandungskante ab. Der Gehäusekörper (100) weist des Weiteren einen ersten Flüssigkeitskanal (600) und einen zweiten Flüssigkeitskanal (700) auf, die beide in Strömungsverbindung mit der Kammer (200) stehen. Im vorliegenden Ausführungsbeispiel ist der Gehäusekörper ein im Wesentlichen starres Spritzgussteil aus Kunststoff. Die Kammer (200), der erste Flüssigkeitskanal (600) und der zweite Flüssigkeitskanal (700) sind jeweils gegenüber einer Umgebung der medizinischen Flüssigkeitskassette halbseitig offen und werden bei der Fertigung der medizinischen Flüssigkeitskassette durch die Abdeckung (300), in vorliegenden Ausführungsbeispiel eine flexible Folie, gegenüber der Umgebung der medizinischen Flüssigkeitskassette abgeschlossen. Die flexible Folie wird dabei im vorliegenden Ausführungsbeispiel zumindest entlang der Umrandungskante (800) mit dem Gehäusekörper (100) verschweißt, beispielsweise mittels Laserschweißen. In der **Figur 1** ist die Abdeckung (300) nur durch das Bezugszeichen angedeutet und entlang der Freihandlinie aufgeschnitten, so dass die Kammer (200), der erste Flüssigkeitskanal (600) und der zweite Flüssigkeitskanal (700) sichtbar sind. Der erste Wandungsabschnitt (110) der Kammer (200) weist eine Aufnahme (500), im vorliegenden Ausführungsbeispiel eine Nut auf, die zum Einstecken oder Einschieben eines Einsatzes (400) vor dem Verschweißen der Abdeckung (300) mit dem Gehäusekörper (100) ausgebildet ist. Der Einsatz (400) selbst wird in **Figur 1** nicht gezeigt. Der Einsatz (400), gezeigt in **Figur 3****,** ist seinerseits ausgebildet, um in die Aufnahme (500) eingeschoben zu werden, solange der Gehäusekörper (100) noch ohne Abdeckung (300) ist. Zu diesem Zweck weist der Einsatz (400) einen ersten Umfangsabschnitt (410) auf, der an die Kontur des ersten Wandungsabschnitts (110) und der Aufnahme (500) angepasst ist. Des Weiteren weist der Einsatz (400) einen zweiten Umfangsabschnitt (420) auf, der ausgebildet ist zum flüssigkeitsdichten Kontakt mit der Abdeckung (300), wenn der Gehäusekörper durch die Abdeckung (300) abgedeckt ist und die Abdeckung (300) an den zweiten Umfangsabschnitt (420) angepresst wird. Eine Anpressung der Abdeckung (300) erfolgt erst während der Behandlung einer medizinischen Flüssigkeit in einer Behandlungsmaschine, also bei der Benutzung der medizinischen Flüssigkeitskassette. Vor der Benutzung der medizinischen Flüssigkeitskassette liegt die Abdeckung (300) lose auf dem Umfangsabschnitt (420) auf. Es ist allerdings in anderen Ausführungsformen auch möglich, die Abdeckung (300) bei der Fertigung der medizinischen Flüssigkeitskassette (1) flüssigkeitsdicht mit dem Umfangsabschnitt (420) zu verbinden, beispielsweise durch Verschweißen oder Verkleben.

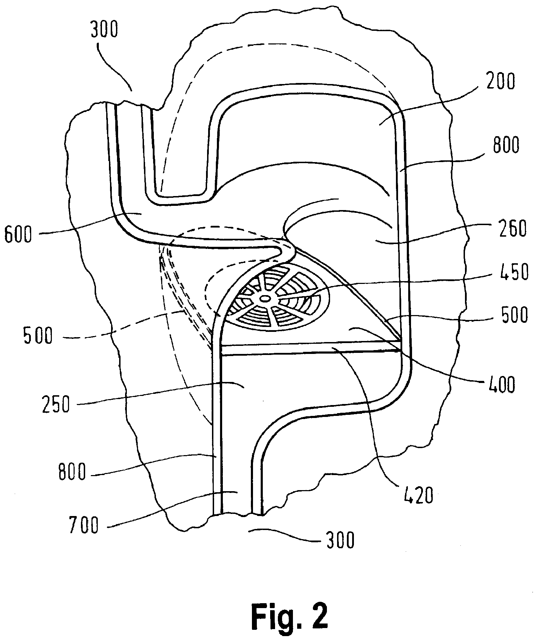

Die **Figur 2** zeigt eine freigeschnittene perspektivische Ansicht der medizinischen Flüssigkeitskassette gemäß **Figur 1****,** jedoch mit eingesetztem Einsatz (400), wobei die Abdeckung (300) wie bereits in **Figur 1** dargestellt in der **Figur 2** wiederum nur durch das Bezugszeichen angedeutet und entlang der Freihandlinie aufgeschnitten ist, so dass die Kammer (200), der erste Flüssigkeitskanal (600) und der zweite Flüssigkeitskanal (700) und Umfangsabschnitt (420) sichtbar sind. Der Umfangsabschnitt (410) ist flüssigkeitsdicht in die als umlaufende Nut ausgeführte Aufnahme (500) eingesetzt. Der Einsatz (400) weist einen Gerinnselfilter (450) auf, der den ersten Kammerabschnitt (250) flüssigkeitsdurchlässig mit dem zweiten Kammerabschnitt (260) verbindet. Der Gerinnselfänger (450) ist in der gezeigten Ausführungsform rund dargestellt.

In anderen besonders bevorzugten Ausführungsformen kann der Gerinnselfänger (450) im Wesentlichen oder vollständig die gesamte Fläche des Einsatzes (400) ausfüllen. Dadurch kann die vom Blut durchströmte Querschnittsfläche des Gerinnselfängers (450) und/oder des Einsatzes (400) maximiert und das Entlüften beim Befüllen mit medizinischer Flüssigkeit sowie das Ablösen von Luftblasen von dem Gerinnselfänger (450) und/oder dem Einsatz (400) begünstigt werden.

Die Kammer (200) im vorliegenden Ausführungsbeispiel ist als Luftblasenabscheider einer Blutkassette für die extrakorporale Blutbehandlung ausgebildet. Vorteile des Luftblasenabscheiders sind das aufgrund der platzsparenden Anordnung des Gerinselfängers (450) große Kammervolumen und der große Strömungsquerschnitt mit besonders beruhigter Strömungsführung insbesondere im ersten Kammerabschnitt (250), der den unteren Bereich des Luftblasenabscheiders ausbildet. Durch die beruhigte Strömungsführung können sich kleinere Luftblasen, die an dem Gerinnselfänger (450) anhaften, zu größeren Luftblasen vereinen, die sich aufgrund ihrer größeren Auftriebskräfte ablösen können. Dadurch wird das Ablösen von Luftblasen von dem Gerinnselfänger (450) und/oder dem Einsatz (400) begünstigt.

Der im vorliegenden Ausführungsbeispiel in den Figuren 1 und 2 gezeigte Ausschnitt einer medizinischen Flüssigkeitskassette ist schematisch. Eine erfindungsgemäße medizinischen Flüssigkeitskassette kann weitere Elemente aufweisen, wie beispielsweise Ventile, Messstellen für Druck- und/oder Temperatur, optische Messstellen, Zuleitungen, Ableitungen sowie weitere Kammern und/oder Kanäle zum Aufnehmen und/oder Führen medizinischer Flüssigkeiten.

In manchen Ausführungsformen weist ein medizinischer Schlauchsatz eine medizinische Flüssigkeitskassette gemäß der vorliegenden Erfindung auf.

In speziellen Ausführungsformen ist ein medizinischer Schlauchsatz mit einer medizinischen Flüssigkeitskassette gemäß der vorliegenden Erfindung als Blutschlauchsatz für die extrakorporale Blutbehandlung ausgebildet.

Die medizinische Flüssigkeitskassette gemäß der vorliegenden Erfindung und/oder ein medizinischer Schlauchsatz mit einer medizinischen Flüssigkeitskassette gemäß der vorliegenden Erfindung sind geeignet und/oder vorgesehen für die Verwendung zum Aufnehmen und/oder Führen einer medizinischen Flüssigkeit.

### Bezugszeichenliste

**Tabelle 1**

| **Bezugszeichen** | **Bezeichnung** |
|---|---|
| 1 | Medizinische Flüssigkeitskassette |
| 100 | Gehäusekörper |
| 110 | erster Wandungsabschnitt |
| 200 | Kammer |
| 220 | zweiter Wandungsabschnitt |
| 250 | ersten Kammerabschnitt |
| 260 | zweiter Kammerabschnitt |
| 300 | Abdeckung |
| 400 | Einsatz |
| 410 | erster Umfangsabschnitt |
| 420 | zweiter Umfangsabschnitt |
| 450 | Öffnungsstruktur |
| 500 | Aufnahme |
| 600 | erster Flüssigkeitskanal |
| 700 | zweiter Flüssigkeitskanal |
| 800 | Umrandungskante |

## Patentansprüche

1. Medizinische Flüssigkeitskassette (1) aufweisend
einen Gehäusekörper (100) der einen ersten Wandungsabschnitt (110) einer Kammer (200) zum Aufnehmen und/oder Führen einer medizinischen Flüssigkeit ausbildet, eine Abdeckung (300), die einen zweiten Wandungsabschnitt (220) der Kammer (200) ausbildet, wobei die Abdeckung zumindest abschnittsweise eine flexible Folie aufweist oder zumindest abschnittsweise eine flexible Folie ist, einen Einsatz (400) mit einem ersten Umfangsabschnitt (410) und einem zweiten Umfangsabschnitt (420), wobei der Einsatz (400) ausgebildet ist zum zumindest teilweisen Ausfüllen eines Querschnitts der Kammer (200), wobei der Querschnitt der Kammer zumindest von dem ersten Wandungsabschnitt (110) und dem zweiten Wandungsabschnitt (220) begrenzt wird, wobei der Einsatz die Kammer in einen ersten Kammerabschnitt (250) und einen zweiten Kammerabschnitt (260) unterteilt, und der erste Kammerabschnitt (250) und/oder der zweite Kammerabschnitt (260) als Luftblasenabscheider ausgebildet ist,
und eine Aufnahme (500) in oder an dem ersten Wandungsabschnitt (110) wobei der erste Umfangsabschnitt (410) in der Aufnahme (500) aufgenommen ist, **dadurch gekennzeichnet, dass** der zweite Umfangsabschnitt (420) ausgebildet ist zum Herstellen eines flüssigkeitsdichten Kontakts mit der flexiblen Folie, wenn die flexible Folie an den zweiten Umfangsabschnitt (420) angepresst ist oder wobei der zweite Umfangsabschnitt (420) mit der flexiblen Folie dauerhaft flüssigkeitsdicht verbunden ist.

2. Medizinische Flüssigkeitskassette gemäß zumindest einem der Ansprüche 1 bis 4, wobei der zweite Umfangsabschnitt (420) gerade ausgeführt ist und wobei der erste Umfangsabschnitt (410) zumindest abschnittsweise gekrümmt oder eckig ausgeführt ist.

3. Medizinische Flüssigkeitskassette gemäß zumindest einem der Ansprüche 1 bis 2, wobei die Aufnahme (500) zumindest abschnittsweise eine Nut oder einen Steg oder einen Absatz aufweist oder daraus gebildet ist.

4. Medizinische Flüssigkeitskassette gemäß zumindest einem der Ansprüche 1 bis 3, wobei der Einsatz (400) zumindest eine Öffnungsstruktur (450) aufweist zum flüssigkeitsdurchlässigen Verbinden des ersten Kammerabschnitts (250) mit dem zweiten Kammerabschnitt (260).

5. Medizinische Flüssigkeitskassette gemäß Anspruch 4, wobei die Öffnungsstruktur (450) einen Gerinnselfilter aufweist.

6. Medizinische Flüssigkeitskassette gemäß zumindest einem der Ansprüche 1 bis 5, wobei die medizinische Flüssigkeitskassette (1) ausgebildet ist zum Messen des Drucks in der medizinischen Flüssigkeit stromaufwärts des Gerinnselfilters und stromabwärts des Gerinnselfilters.

7. Medizinischer Schlauchsatz aufweisend eine medizinische Flüssigkeitskassette gemäß zumindest einem der Ansprüche 1 bis 6.

8. Medizinischer Schlauchsatz gemäß Anspruch 7, wobei der medizinische Schlauchsatz als Blutschlauchsatz für eine extrakorporale Blutbehandlung ausgebildet ist.

## Claims

1. Medical liquid cassette (1), having
a housing body (100), which forms a first wall section (110) of a chamber (200) for receiving and/or carrying a medical liquid, a cover (300), which forms a second wall section (220) of the chamber (200), wherein the cover has a flexible film in at least some sections or is a flexible film in at least some sections,
an insert (400) with a first peripheral section (410) and a second peripheral section(420), wherein the insert (400) is designed for at least partially filling a cross section of the chamber (200),
wherein the cross section of the chamber is delimited at least by the first wall section (110) and the second wall section (220), wherein the insert divides the chamber into a first chamber section (250) and a second chamber section (260), and the first chamber section (250) and/or the second chamber section (260) is designed as an air bubble separator,
and a seat (500) in or on the first wall section (110), wherein the first peripheral section (410) is received in the seat (500),
**characterized in that** the second peripheral section (420) is designed for establishing a liquid-tight contact with the flexible film when the flexible film is pressed onto the second peripheral section (420), or wherein the second peripheral section (420) is connected to the flexible film in a permanently liquid-tight manner.

2. Medical liquid cassette according to at least one of Claims 1 to 4, wherein the second peripheral section (420) is straight, and wherein the first peripheral section (410) is curved or angular in at least some sections.

3. Medical liquid cassette according to at least one of Claims 1 to 2, wherein the seat (500) has in at least some sections a groove or a web or a shoulder or is formed therefrom.

4. Medical liquid cassette according to at least one of Claims 1 to 3, wherein the insert (400) has at least one opening structure (450) for liquid-permeable connection of the first chamber portion (250) to the second chamber portion (260).

5. Medical liquid cassette according to Claim 4, wherein the opening structure (450) has a clot filter.

6. Medical liquid cassette according to at least one of Claims 1 to 5, wherein the medical liquid cassette (1) is designed for measuring the pressure in the medical liquid upstream from the clot filter and downstream from the clot filter.

7. Medical hose set, having a medical liquid cassette according to at least one of Claims 1 to 6.

8. Medical hose set according to Claim 7, wherein the medical hose set is designed as a blood hose set for an extracorporeal blood treatment.

## Revendications

1. Cassette de fluide médical (1) présentant un corps de boîtier (100) qui constitue une première portion de paroi (110) d'une chambre (200) pour recevoir et/ou guider un fluide médical, un recouvrement (300) qui constitue une deuxième portion de paroi (220) de la chambre (200), le recouvrement présentant au moins en partie ou étant au moins en partie une feuille flexible,
un insert (400) avec une première portion périphérique (410) et une deuxième portion périphérique (420), l'insert (400) étant réalisé pour remplir au moins en partie une section transversale de la chambre (200), la section transversale de la chambre étant limitée au moins par la première portion de paroi (110) et la deuxième portion de paroi (220), l'insert divisant la chambre en une première portion de chambre (250) et une deuxième portion de chambre (260), la première portion de chambre (250) et/ou la deuxième portion de chambre (260) étant réalisées sous forme de séparateur de bulles d'air,
et un logement (500) dans ou sur la première portion de paroi (110), la première portion périphérique (410) étant reçue dans le logement (500),
**caractérisée en ce que** la deuxième portion périphérique (420) est réalisée pour établir un contact étanche aux fluides avec la feuille flexible, lorsque la feuille flexible est pressée contre la deuxième portion périphérique (420) ou la deuxième portion périphérique (420) étant reliée de manière étanche aux fluides de manière durable avec la feuille flexible.

2. Cassette de fluide médical selon au moins l'une des revendications 1 à 4, dans laquelle la deuxième portion périphérique (420) est réalisée sous forme droite et dans laquelle la première portion périphérique (410) est réalisée au moins en partie sous forme courbe ou angulaire.

3. Cassette de fluide médical selon au moins l'une des revendications 1 et 2, dans laquelle le logement (500) présente au moins en partie une rainure ou une nervure ou un épaulement ou est formé par celui-ci ou celle-ci.

4. Cassette de fluide médical selon au moins l'une des revendications 1 à 3, dans laquelle l'insert (400) présente au moins une structure d'ouverture (450) pour la connexion perméable aux liquides de la première portion de chambre (250) avec la deuxième portion de chambre (260).

5. Cassette de fluide médical selon la revendication 4, dans laquelle la structure d'ouverture (450) présente un filtre à caillots.

6. Cassette de fluide médical selon au moins l'une des revendications 1 à 5, la cassette de fluide médical (1) étant réalisée pour mesurer la pression dans le fluide médical en amont du filtre à caillots et en aval du filtre à caillots.

7. Jeu de tuyaux médicaux présentant une cassette de fluide médical selon au moins l'une des revendications 1 à 6.

8. Jeu de tuyaux médicaux selon la revendication 7, le jeu de tuyaux médicaux étant réalisé sous forme de jeu de tuyaux de sang pour un traitement extracorporel du sang.
